## Europäisches Patentamt

## European Patent Office

## Office européen des brevets

(11) Veröffentlichungsnummer: **0 039 048**
**A1**

(12) **EUROPÄISCHE PATENTANMELDUNG**

(21) Anmeldenummer: 81103057.6

(22) Anmeldetag: 23.04.81

(51) Int. Cl.³: **C 07 C 55/02**
C 07 C 55/14, C 07 C 55/22
C 07 C 55/24, C 07 C 51/487
C 07 C 51/31, C 07 C 51/47

(30) Priorität: 26.04.80 DE 3016225

(43) Veröffentlichungstag der Anmeldung:
04.11.81 Patentblatt 81.44

(84) Benannte Vertragsstaaten:
CH DE FR GB IT LI NL

(71) Anmelder: Ruhrchemie Aktiengesellschaft
Bruchstrasse 219
D-4200 Oberhausen 13(DE)

(72) Erfinder: Bexten, Ludger, Dr. Dipl.-Chem.
im Freihof 9
D-4224 Hünxe(DE)

(72) Erfinder: Brundin, Eike, Dr. Dipl.-Chem.
Voerder Strasse 127
D-4220 Dinslaken(DE)

(74) Vertreter: Reichelt, Karl-Heinz, Dr.
m. Br. Ruhrchemie Aktiengesellschaft Abt. PLD Postfach
13 01 60
D-4200 Oberhausen 13(DE)

(54) Verfahren zur Reinigung von Polycarbonsäuren.

(57) Stickstoffhaltige Verbindungen enthaltende Polycarbonsäuren, die durch Oxidation cyclischer Alkene mit Salpetersäure erhalten wurden, reinigt man erfindungsgemäß durch oxidative Nachbehandlung. Besonders geeignete Oxidationsmittel für diese Nachbehandlung sind Salpetersäure oder Wasserstoffperoxid. Anschließend an den Reinigungsschritt werden die Polycarbonsäuren insbesondere mit Wasser oder mit $C_2$- bis $C_8$-Carbonsäuren gewaschen.

EP 0 039 048 A1

Ruhrchemie Aktiengesellschaft, Oberhausen 13

Verfahren zur Reinigung von Polycarbonsäuren

Die Erfindung betrifft ein neues Verfahren zur Reinigung von zwei oder mehrbasigen Polycarbonsäuren, die durch Oxidation cyclischer Alkene und Alkenderivate mit 5 bis 16 C-Atomen, die mindestens eine C=C-Doppelbindung im Ring enthalten, mit Salpetersäure bei 40 bis 70 $^{\circ}$C in Gegenwart von Oxidationskatalysatoren hergestellt worden sind. Polycarbonsäuren haben technische Bedeutung u.a. für die Herstellung von Polyestern, Polyamiden und Polyimiden erlangt.

Es ist bekannt, durch Oxidation cyclischer Alkene mit Salpetersäure unter Ringaufspaltung $\omega\omega'$-Dicarbonsäuren zu bilden. So erhält man nach einer in der US-PS 23 23 861 beschriebenen Arbeitsweise aus Cyclohexen und Cyclohexenderivaten mit 30 bis 75 %-igerSalpetersäure Adipinsäure bzw. die entsprechenden Adipinsäurederivate. Die maximale Reaktionstemperatur wird mit 70 bis 90 $^{\circ}$C angegeben. Als Katalysator findet u.a. Ammoniumvanadat Verwendung.

Ein wesentlicher Nachteil der bekanntenOxidationsverfahren stellt die Verunreinigung der Reaktionsprodukte durch Nitroverbindungen dar. Nach der US-PS 38 39 377

0039048

R 1888

oxidiert man 1-Olefine mit Salpetersäure unterhalb 65 °C und vollendet die Oxidation im Temperaturbereich von 75 bis 110 °C. Hierbei steigt zwar die Carbonsäure-Ausbeute von 79 bis 85 Gew.-% auf 85 bis 89,5 Gew.-% an, gleichzeitig erhöht sich jedoch auch der Anteil der unerwünschten Stickstoffverbindungen von 2 bis 5 Gew.-% auf 4 bis 15 Gew.-%. Die so erhaltenen Carbonsäuren werden daher aus dem Oxidationsrohprodukt mittels wäßriger Lauge extrahiert und so von den störenden Stickstoffverbindungen befreit. Ebenfalls als schädlich wird die Anwendung von Temperaturen oberhalb 70 °C bei der Salpetersäure-Oxidation von Cyclohexen bzw. Cyclohexen-Derivaten angesehen (John E. Franz, John F. Herber, William F. Knowles; J.Organ.Chem. 30, 1488 (1965)). Die Autoren fanden, daß diese Temperaturen bei der Salpetersäure-Oxidation cyclischer Alkene, wie Cyclohexen und Tetrahydrophthalsäure, eine drastische Minderung der Ausbeute an Carbonsäure zur Folge haben. So erhält man bei der Oxidation von $\Delta^4$ - Tetrahydrophthalsäure im Temperaturbereich von 55 bis 60 °C 1,2,3,4-Butantetracarbonsäure in 75 %-iger Ausbeute. Erhöht man die Temperatur auf 90 °C, so verringert sich die Ausbeute auf unterhalb 35 %.

Wegen der Bildung von stickstoffhaltigen Verbindungen ist es unerläßlich, die bei der Salpetersäure-Oxidation cyclischer Alkene gebildeten Carbonsäuren in einem nachfolgenden Reinigungsschritt auf das Reinprodukt aufzuarbeiten. Dies erfolgt in der Regel entweder durch Sublimation oder Kristallisation. Beide Arbeitsweisen sind technisch recht aufwendig und mit nicht unbeträchtlichen Produktverlusten verbunden.

Es bestand daher die Aufgabe, ein Verfahren zu entwickeln, das diese Nachteile vermeidet. Überraschend führt das erfindungsgemäß beanspruchte Verfahren zur Reinigung von 2 oder mehrbasischen Polycarbonsäuren, die durch Oxidation cyclischer Alkene und Alkenderivate mit 5 bis 16 C-Atomen, die mindestens eine C=C-Doppelbindung im Ring enthalten, mit Salpetersäure bei 40 bis 70 $^{o}$C in Gegenwart von Oxidationskatalysatoren hergestellt worden sind, dadurch gekennzeichnet, daß die nach Oxidation anfallenden Polycarbonsäuren oxidativ nachbehandelt werden. Überraschend führt das erfindungsgemäße Verfahren ohne Minderung der Ausbeute direkt zu reinen Polycarbonsäuren, die nicht in einem separaten Reinigungsschritt wie Sublimation oder Kristallisation nachgereinigt werden müssen.

Das neue Verfahren kann in Sonderfällen für sich alleine zur Reinigung von Polycarbonsäuren benutzt werden, es kann aber auch als Vorreinigungsstufe dienen, wobei es dann den weiteren Reinigungsprozeß vereinfacht und unterstützt.

Als cyclische Alkene und Alkenderivate werden Cyclopenten, Cyclohexen, 3-Cyclohexencarboxaldehyd, 3-Cyclohexencarbonsäure, 2-Methyl-4-cyclohexencarbonsäure, Tetrahydrophthalsäure, Tetrahydrophthalsäureanhydrid, 2-Phenyl-4-cyclohexencarbonsäure und 3,6-Methylen-$\Delta^{4}$-tetrahydrophthalsäure und deren Anhydrid eingesetzt.

- 4 -

0039048

R 1888

Die oxidative Nachbehandlung wird mit Salpetersäure oder Wasserstoffperoxid gegebenenfalls in Gegenwart von Katalysatoren durchgeführt. Dadurch werden sowohl stickstoffhaltige Nebenprodukte als auch thermisch instabile, verfärbende Verunreinigungen abgebaut. Die oxidative Nachbehandlung erfolgt bei erhöhten Temperaturen von 90 bis 150 $^{o}$C - insbesondere bei Temperaturen von 90 bis 120 $^{o}$C. Die weitere Aufarbeitung der Polycarbonsäuren erfolgt nach der oxidativen Nachbehandlung durch Waschen. Bei der Wäsche des Produktes werden entweder Wasser oder Carbonsäuren mit 2 bis 8 C-Atomen verwendet. Die eingesetzte Carbonsäure des Waschvorganges wird mittels Reduktionsmitteln wie Ameisensäure von Salpetersäure oder Wasserstoffperoxid befreit. Wasserstoffperoxid kann auch durch Verkochen entfernt werden. Nach Reinigung wird die Carbonsäure des Waschvorganges redestilliert und kann erneut zur Wäsche eingesetzt werden.

Nach dem erfindungsgemäßen Prozeß erhält man Polycarbonsäuren, die frei von stickstoffhaltigen Verbindungen und thermisch instabilen, verfärbenden Verunreinigungen sind. Die Polycarbonsäuren können ohne weitere Reinigung, wie Sublimation oder Kristallisation weiterverarbeitet werden. Selbst bei den hohen Temperaturen, wie bei der Esterherstellung erforderlich, verfärben sie sich nicht.

Beispiel 1 (Vergleichsversuch)

In einem mit Rührwerk und Abgaskühler bestückten 5 l-Glasreaktor werden 0,5 g Ammoniumvanadat und 2700 g Abfallsalpetersäure (18,8 Gew.-% $HNO_3$) vorgelegt. Die Abfallsalpetersäure entstammt aus vorangegangenen Versuchen. Bei einer Reaktionstemperatur von 60 bis 65 °C werden 713 g $\Delta^4$-Tetrahydrophthalsäureanhydrid und 1860 g 65-%ige Salpetersäure innerhalb von 2 Stunden in den Reaktor eingebracht. Zur Vervollständigung der Oxidation wird der Reaktorinhalt auf 70 °C erwärmt und die klare Lösung noch 4 Stunden lang bei 70 °C gerührt. Beim Abkühlen auf Raumtemperatur kristallisiert die Butantetracarbonsäure aus. Das Kristallisat wird abfiltriert und dreimal mit je 1000 ml Essigsäure gewaschen und im Trockenschrank (Wasserstrahlvakuum) bei 70 °C getrocknet.

Es werden 739 g 1,2,3,4-Butantetracarbonsäure erhaten, daneben fallen 3310 g Abfallsäure an.

Die 1,2,3,4-Butantetracarbonsäure ist frei von Salpetersäure.
Stickstoffgehalt: 0,5 Gew.-%.
Verfärbungstest : 20 g Butantetracarbonsäure werden in 80 g Ethylenglykol für 30 Minuten auf 140 °C erhitzt, hierbei verfärbt sich die Lösung dunkelbraun.
Farbzahl der Lösung:   1000 (mgJod/100 ml).

0039048

**Beispiel 2** (oxidative Nachbehandlung)

Unter Benutzung der gleichen Abfallsalpetersäure wie in Beispiel 1, wird die Oxidation von $\Delta^4$-Tetrahydrophthalsäureanhydrid, wie in Beispiel 1 angegeben, durchgeführt. Zur Vervollständigung der Oxidation wird der Reaktorinhalt auf 70 °C erwärmt und die klare Lösung 3 Stunden lang bei 70 °C gerührt. Im Anschluß hieran wird die Lösung auf 90 °C erhitzt und noch 1 Stunde lang bei 90 °C unter Rühren oxidativ nachbehandelt. Die weitere Aufarbeitung erfolgt wie in Beispiel 1. Es werden 730 g Butantetracarbonsäure erhalten, daneben fallen 3278 g Abfallsalpetersäure an. Die Butantetracarbonsäure ist frei von Salpetersäure.

Stickstoffgehalt: $< 0,01$ Gew.-%.

Verfärbungstest :      20 g Butantetracarbonsäure werden in 80 g Ethylenglykol für 30 Minuten auf 140 °C erhitzt, hierbei tritt keine Verfärbung ein.

Farbzahl der Lösung:  1 (mgJod/100 ml).

**Beispiel 3** (Vergleichsversuch)

In einem mit Rührwerk und Abgaskühler bestückten 5 l Glasreaktor werden 1000 g 30 %-ige Salpetersäure, 2 g Ammoniumvanadat und 10 g Natriumnitrit vorgelegt. Bei einer Reaktionstemperatur von 40 bis 45 °C werden 205 g Cyclohexen und 1100 g 65 %ige Salpetersäure innerhalb von 4 Stunden in den Reaktor eingebracht.

0039048

Zur Vervollständigung der Oxidation wird der Reaktorinhalt noch 1 Stunde bei 50 °C, 1 Stunde bei 60 °C und 6 Stunden bei 70 °C gerührt. Beim Abkühlen auf Raumtemperatur kristallisiert die Addipinsäure aus. Das Kristallisat wird abfiltriert, dreimal mit je 500 ml Wasser gewaschen und im Trockenschrank (Wasserstrahlvakuum) bei 70 °C getrocknet. Es werden 181 g Addipinsäure erhalten. Die Addipinsäure ist frei von Salpetersäure.

Stickstoffgehalt: 0,29 Gew.-%

Die Addipinsäure wird in 500 ml Wasser heiß gelöst, die heiße Lösung zur Abtrennung öliger Verunreinigungen filtriert und anschließend durch Abkühlen kristallisiert. Dieser Vorgang wird ohne zu Filtrieren einmal wiederholt.

Verfärbungstest: Nach Umkristallisieren werden 20 g Addipinsäure in 80 g Ethylenglykol für 30 Minuten auf 140 °C erhitzt.

Farbzahl der Lösung: 7 (mg Jod/100 ml).


Beispiel 4 (oxidative Nachbehandlung)

Cyclohexen wird, wie in Beispiel 3 angegeben, oxidiert. Zur Vervollständigung der Oxidation wird der Reaktorinhalt noch eine Stunde bei 50 °C, eine Stunde bei 60 °C und eine Stunde bei 70 °C gerührt. Im Anschluß hieran wird auf 90 °C erhitzt und noch eine Stunde lang bei 90 °C unter Rühren oxidativ nachbehandelt. Beim Abkühlen auf Raumtemperatur kristallisiert die Adipinsäure aus. Das Kristallisat wird abfiltriert und dreimal mit je 500 ml Wasser gewaschen und im Trockenschrank (Wasserstrahlvakuum) bei 70 °C getrocknet.

Es werden 180 g Addipinsäure erhalten. Die Addipinsäure ist frei von Salpetersäure.
Stickstoffgehalt: 0,03 Gew.-%.
Verfärbungstest: 20 g Addipinsäure werden in 80 g Ethylenglykol für 30 Minuten auf 140 $^\circ$C
erhitzt, hierbei tritt keine Verfärbung ein.
Farbzahl der Lösung: < 1 (mg Jod/100 ml).

Beispiel 5 (Vergleichsversuch)
Gemäß der Arbeitsweise in Beispiel 1 wurden an Stelle von
Tetrahydrophthalsäureanhydrid 700 g 3-Cyclohexencarboxaldehyd eingesetzt und verarbeitet. Nach Filtration, Waschung mit Essigsäure und Trocknung werden 752 g 1.2.4-
Butantricarbonsäure erhalten. Die 1.2.4-Butantricarbonsäure
ist frei von Salpetersäure.
Stickstoffgehalt: 0,4 Gew.-%.
Verfärbungstest: 20 g 1.2.4-Butantricarbonsäure werden
in 80 g Ethylenglykol für 30 Minuten
auf 140 $^\circ$C erhitzt, hierbei verfärbt
sich die Lösung dunkelbraun.
Farbzahl der Lösung: > 1000 (mg Jod/100 ml).

Beispiel 6 (oxidative Nachbehandlung)
An Stelle von Tetrahydrophthalsäureanhydrid werden 700 g
3-Cyclohexencarboxaldehyd, wie in Beispiel 2 angegeben,
eingesetzt und verarbeitet. Nach Filtration, Waschung
mit Essigsäure und Trocknung fallen 747 g 1.2.4-Butantricarbonsäure an. Die 1.2.4-Butantricarbonsäure ist
frei von Salpetersäure.
Stickstoffgehalt: < 0,01 Gew.-%.

0039048

Verfärbungstest:    20 g 1.2.4-Butantricarbonsäure werden
in 80 g Ethylenglykol für 30 Minuten
auf 140 °C erhitzt, hierbei tritt keine
Verfärbung ein.

Farbzahl der Lösung: < 1 (mg Jod/100 ml).

Beispiel 7 (oxidative Nachbehandlung)
100 g Butantetracarbonsäure (nicht oxidativ nachbehandeltes Produkt aus Beispiel 1) werden in 100 g Wasser und 30 g Perhydrol (30 %-ig $H_2O_2$) unter Erwärmen gelöst. Die intensiv gefärbte gelbe Lösung wird 2 Stunden lang unter Sieden erhitzt, wobei sich die Lösung deutlich aufhellt. Beim Abkühlen auf Raumtemperatur kristallisiert die Butantetracarbonsäure aus. Das Kristallisat wird abfiltriert und dreimal mit je 140 ml Essigsäure gewaschen. Die Butantetracarbonsäure ist frei von Salpetersäure.
Stickstoffgehalt: 0,03 Gew.-%.
Verfärbungstest:    20 g Butantetracarbonsäure werden in
80 g Ethylenglykol für 30 Minuten auf
140 °C erhitzt, hierbei tritt keine
Verfärbung ein.
Farbzahl der Lösung: < 1 (ml Jod/100 ml).

0039048

Oberhausen, 23.04.1980
PLD bin-sei - R 1888 -

Ruhrchemie Aktiengesellschaft, Oberhausen 13

## Patentansprüche

1.) Verfahren zur Reinigung von zwei- oder mehrbasischen Polycarbonsäuren, die durch Oxidation cyclischer Alkene und Alkenderivate mit 5 bis 16 C-Atomen, die mindestens eine C = C Doppelbindung im Ring enthalten, mit Salpetersäure bei 40 bis 70°C in Gegenwart von Katalysatoren hergestellt worden sind, dadurch gekennzeichnet, daß die nach Oxidation anfallenden Polycarbonsäuren oxidativ nachbehandelt werden.

2.) Verfahren nach Anspruch 1, dadurch gekennzeichnet, daß als cyclische Alkene und Alkenderivate Cyclopenten, Cyclohexen, 3-Cyclohexencarboxaldehyd, 3-Cyclohexencarbonsäure, 2-Methyl-4-cyclohexencarbonsäure, Tetrahydrophthalsäure, Tetrahydrophthalsäureanhydrid, 2-Phenyl-4-cyclohexencarbonsäure und 3,6-Methylen- $\Delta^4$-tetrahydrophthalsäure und deren Anhydrid eingesetzt werden.

3.) Verfahren nach Anspruch 1 und 2, dadurch gekennzeichnet, daß die oxidative Nachbehandlung mit Salpetersäure oder Wasserstoffperoxid gegebenenfalls in Gegenwart von Katalysatoren erfolgt.

4.) Verfahren nach Anspruch 1 bis 3, dadurch gekennzeichnet, daß die oxidative Nachbehandlung bei Temperaturen von 90 bis 150°C - insbesondere bei Temperaturen von 90 bis 120°C - erfolgt.

R 1888

5.) Verfahren nach Anspruch 1 bis 4, dadurch gekennzeichnet, daß die nach oxidativer Nachbehandlung anfallenden Polycarbonsäuren durch Waschen gereinigt werden.

6.) Verfahren nach Anspruch 1 bis 5, dadurch gekennzeichnet, daß die Wäsche der Polycarbonsäuren mit Wasser oder mit $C_2$- bis $C_8$-Carbonsäuren erfolgt.

7.) Verfahren nach Anspruch 6, dadurch gekennzeichnet, daß die zur Wäsche verwendeten Carbonsäuren vom Oxidationsmittelüberschuß befreit und anschließend destilliert werden.

**0039048**
Nummer der Anmeldung

EP 81 10 3057

| Europäisches Patentamt | **EUROPÄISCHER RECHERCHENBERICHT** |
|---|---|

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 2 218 314 (SOCIETE NATIONALE DES PETROLES D'AQUITAINE) <br> * Seite 2, Zeile 8 - Seite 5 * | 1,3-6 |
| | -- | |
| X | FR - A - 876 620 (F. RASCHIG) <br> * Beispiel 2 * | 1-4 |
| | -- | |
| | DE - A - 1 919 228 (CHEMISCHE WERKE HÜLS) <br> * Seite 3, Zeile 24 - Seite 4, Zeile 10; Beispiele 1,2; Anspruch 1 * | 1,3 |
| | -- | |
| | US - A - 2 703 331 (M. GOLDBECK et al.) <br> * Anspruch 1 * | 1,3,4 |
| | -- | |
| | FR - A - 2 092 524 (MITSUBISHI) <br> * Beispiel 1; Ansprüche 1-6 * | 1,3-5 |
| | -- | |
| | DE - B - 1 289 038 (HALCON INT.) <br> * Beispiele 1,2; Anspruch 1 * | 5-7 |
| | -- | |
| A | FR - A - 1 331 267 (ESSO) <br> * Beispiel 3; Ansprüche * | 1 |
| | -- | |
| A | US - A - 3 461 160 (E.D. WILHOIT) <br> * Beispiel 1; Ansprüche * | 1,2 |
| | ---- | |

### KLASSIFIKATION DER ANMELDUNG (Int Cl.)

C 07 C 55/02
55/14
55/22
55/24
51/487
51/31
51/47

### RECHERCHIERTE SACHGEBIETE (Int Cl)

C 07 C 55/02
55/12
55/14
55/22
55/24
51/42
51/487
51/31

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 26-06-1981 | LI VOTI |

EPA form 1503.1 06.78